Europäisches Patentamt

European Patent Office

Office européen des brevets

(19)

(11) Publication number: **0 112 812**
**A2**

(12) # EUROPEAN PATENT APPLICATION

(21) Application number: **83870135.7**

(22) Date of filing: **19.12.83**

(51) Int. Cl.³: **C 12 M 1/12**

(30) Priority: **20.12.82  US 450836**

(43) Date of publication of application: **04.07.84**
**Bulletin 84/27**

(84) Designated Contracting States: **CH DE GB LI SE**

(71) Applicant: **MONSANTO COMPANY, Patent Department 800 North Lindbergh Boulevard, St. Louis, Missouri 63166 (US)**

(72) Inventor: **Bild, Gary Stephen, 9 Blackpool Lane, Olivette Missouri 63123 (US)**
Inventor: **Glover, George Irvin, 22 Wind Rush Creek West, Creve Coeur Missouri 63141 (US)**

(74) Representative: **Lunt, John Cooper et al, Monsanto Europe S.A. Patent Department Avenue de Tervuren 270-272 Letter Box No 1, B-1150 Brussels (BE)**

(54)    **Biocatalytic reactor.**

(57) Apparatus for conducting continuous biocatalytic reactions comprises a first zone (23) and a second zone (17) separated each from the other at least in part by a semipermeable membrane. In use, the membrane is permeable to predetermined reactant and product materials while impermeable to catalytically active microbial cell material essentially confluently packed within the second zone (17). The reactant material supplied to the first zone (23) permeates the membrane and contacts catalyst in the second zone (17) thereby forming product material. Product material permeates the membrane and is removed from the first zone (23).

BIOCATALYTIC REACTOR

BACKGROUND OF THE INVENTION

This invention relates to a novel process and apparatus for carrying out continuous enzymatic reactions. More specifically, this invention provides an improved immobilized microbial cell biocatalytic reactor.

Enzymes are protein catalysts which are used in many research and commercial applications. Enzymes are highly specific in reaction catalyzed and, therefore, generally yield only small amounts of by-products. Enzymatic reactions are attractive for commercial processing since these reactions generally require mild reaction conditions and often have high conversion levels to desired products. Enzymatic reactions can be easily effected by admixing enzyme and substrate in a batch reactor. Unfortunately, this type of processing generally has an attendant loss of enzyme during product recovery.

To circumvent this problem, various methods have been developed to immobilize or bind enzymes to insoluble carriers. Generally, enzyme immobilization can be accomplished by covalent bonding, ionic bonding, physical adsorption, cross-linking, encapsulation within a lattice structure, and microencapsulation. For example, U.S. Patent 4,033,817 discloses a process for effecting enzymatic reactions wherein an activator is forced through a nonanisotropic ultrafiltration membrane under pressure to form active sites. Enzyme is then forced through the activated membrane under pressure thereby chemically coupling enzyme to the activated membrane. The enzymatic reaction is effected by forcing reactant material through the enzyme-coupled membrane.

Hollow fiber semipermeable membranes have been used to construct immobilized enzyme reactors. Enzyme catalyst has been immobilized in the macroporous sponge layer of anisotropic hollow fibers by soaking the fibers in saturated enzyme solution, Westerland et al., AIChE Journal, 20 (1), PP. 50-59 (1974). Alternately, purified enzyme catalyst can be placed in the dialyzer shell outside the fiber; Davis, Biotechnology and Bioengineering, Vol. XVI, pp.1113-1122 (1974). U.S. Patent 4,266,026 discloses a process wherein catalyst, preferably enzyme, is encapsulated in the sponge layer of anisotropic fibers. Catalyst is entrapped by flushing catalyst from the sponge side into the fiber lumen under a pressure gradient. The enzyme can be further secured by cross-linking the enzyme to the sponge layer. A disadvantage shared by most enzyme immobilization methods is that the enzyme is typically isolated and purified before immobilization.

In order to eliminate enzyme isolation, various methods of microbial cell immobilization have been developed. These methods usually involve either adsorption onto insoluble carriers, encapsulation in liquid-surfactant membranes, and entrapment in polyacrylamide gel, collagen, or agar. For example, U.S. Patent 3,875,008 discloses a method wherein enzyme or microbial cells are encapsulated in hollow fiber filaments by extruding a polymer solution through an orifice while at the same time injecting a solution of enzyme or microbial cells through the internal portion of the orifice. This method is costly since the fiber/cell composite must be extruded for each individual enzyme or microbial species desired. Moreover, spent microbial cells cannot be easily replaced since the cells are entrapped within the lumen of the fiber.

Whole cell immobilization in a hollow fiber dialyzer has been described by Kan and Shuler, Biotechnology and Bioengineering, Vol. XX, pp. 217-230, (1978). In the Kan and Shuler disclosure, heat-treated Pseudomonas fluorescens cells (strain ATCC 11299b) are immobilized in a hollow fiber dialyzer cartridge to produce urocanic acid from L-histidine. Cells are cultured, harvested, concentrated, and heat-treated outside the dialyzer unit. The resulting viscous cell suspension is then poured into the shell of the dialyzer unit. This method requires extensive handling and preparation of the microbial cell culture prior to packing the dialyzer unit. The cell concentration is limited since the viscous suspension must be poured into the shell-side of the dialyzer. Moreover, only 70% of the shell-side capacity could be used due to the viscous nature of the cell suspension.

It is, therefore, the overall object of the present invention to provide an improved process and apparatus for carrying out continuous biocatalytic reactions.

Accordingly, it is an object of the present invention to provide a biocatalytic reactor apparatus which significantly enhances the stability of biochemical reaction systems.

It is another object of the present invention to provide a biocatalytic reactor apparatus wherein the catalytic zone is essentially confluently packed with catalytically active microbial cell material.

It is a further object of the present invention to achieve the highest possible catalyst concentration by packing viable microbial cells which multiply within the catalytic zone during the packing procedure.

It is still another object of the present invention to provide a biocatalytic reactor apparatus wherein microbial cells are essentially confluently packed within the catalytic zone such that the cells adhere tightly to the semipermeable membrane thereby eliminating the need to chemically or physically attach cells to said membrane.

It is yet another object of the present invention to provide an improved apparatus wherein spent microbial cells can be easily flushed from the catalytic zone and replaced with fresh microbial cells.

It is still a further object of the present invention to provide an apparatus that can utilize either anisotropic or nonanisotropic semipermeable membranes.

These and other objects, features, and advantages of the present invention will become apparent to those skilled in the art from the following description and figures.

SUMMARY OF THE INVENTION

It has now been discovered that an improved biocatalytic reactor can be constructed by immobilizing catalytically active microbial cell material within the shell of a semipermeable membrane dialyzer in concentrations far exceeding that reported heretofore. While not fully understood, the unexpected and surprising enzyme stabilizing feature for some enzyme systems appears to result from the essentially confluent cellular packing within the catalytic zone.

The dialyzer shell volume is essentially confluently packed with catalytically active microbial cell material to form a catalytic zone. The shell ports are then closed. A predetermined reaction is carried out by supplying reactant material to the reactant/product zone. Product material is then recovered from the effluent of said zone.

It should be understood that the following preferred embodiment utilizing a hollow fiber ultrafiltration dialyzer is not intended to limit the scope of the invention. One skilled in the art can make various structural modifications while not departing from the spirit of the present invention.

BRIEF DESCRIPTION OF THE DRAWING

Figure 1 is a longitudinal, cross-sectional view of a hollow fiber dialyzer. When the dialyzer shell volume is essentially confluently packed with catalytically active microbial cell material in accordance with the present invention, said dialyzer unit becomes a biocatalytic reactor.

Figure 2 is an axial, cross-sectional view of a hollow fiber dialyzer unit suitable for use in the present invention.

Figure 3 is a simplified schematic representation of an embodiment suitable for the practice of the present invention.

DESCRIPTION OF THE PREFERRED EMBODIMENT

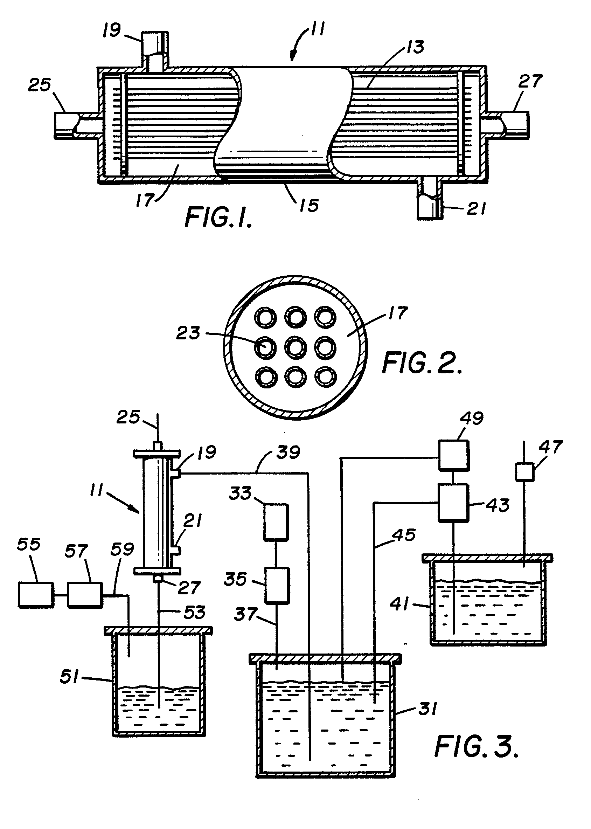

Figures 1 and 2 illustrate a hollow fiber dialyzer unit suitable for use in the present invention. Hollow fiber dialyzer unit 11 is like in structure to a single-pass shell and tube heat exchanger wherein a multiplicity of hollow fiber membranes 13 are juxtaposed essentially axially within cylindrical shell 15. Catalytically active microbial cell material is confluently packed in shell-side volume 17 to form the catalytic zone. Access to shell-side volume 17 is provided by inlet port 19 and outlet port 21. Lumen-side volume 23 functions as the reactant/product zone. Access to lumen-side volume 23 is provided by inlet port 25 and outlet port 27.

Although the preferred embodiment described herein comprises the use of a hollow fiber dialyzer unit, it is not intended to limit the scope of the invention. It should be understood that the present invention can be successfully practiced with essentially any semipermeable membrane dialyzer which permits transfer of reactant and product across the membrane while substantially prohibiting the transfer of catalytically active microbial cell material.

It should be further understood that although the preferred embodiment described herein comprises the use of whole microbial cells, it is not intended to limit the scope of the invention. Rather, it should be understood that the present invention can be successfully practiced with catalytically active microbial cell material such as lysed microbial cells. One skilled in the art may choose to lyse the cells prior to or after packing in order to free intracellular enzyme.

Referring to Figure 3, microbial cells are grown in liquid culture vessel 31. Culture vessel 31 is maintained in slight positive pressure by an aseptic air source 33 through regulator 35 and conduit 37. Microbial cells are transferred from culture vessel 31 to shell-side inlet port 19 of hollow fiber cartridge 11 under said pressure by conduit 39. Volume withdrawn from culture vessel 31 is replaced with fresh culture medium from medium reservoir 41 by pump 43 through conduit 45. Medium reservoir 41 is vented through filter 47. Liquid level in culture vessel 31 is maintained by liquid level controller 49. While loading microbial cells into hollow fiber cartridge 11, shell-side outlet port 21 and lumen-side inlet port 25 are closed. Lumen-side outlet port 27 is connected to culture filtrate collection vessel 51 by conduit 53. Culture filtrate vessel 51 is maintained in slight negative pressure by vacuum source 55 through regulator 57 and conduit 59.

Cell suspension is transferred from culture vessel 31 through shell-side inlet port 19 of hollow fiber cartridge 11. Culture filtrate diffuses across the hollow fibers due to the pressure gradient across the fiber membrane while microbial cells remain within shell-side volume 17 of hollow fiber cartridge 11. Cell packing continues until the entire shell-side volume 17 is densely packed with microbial cells forming a catalytic zone. For purposes of the present invention, the dense cellular packing is such that said microbial cells are essentially confluently packed throughout the shell-side volume. Microbial cells are confluently packed when adjacent cells are adjoined so that the interstitial space between adjacent cells is minimized. More specifically, said microbial cells occupy greater than about 50 percent of the catalytic zone utilized and preferably greater than about 90 percent. Culture filtrate is collected into culture filtrate vessel 51. Inlet port 19 is closed when the shell-side volume 17 is confluently packed with microbial cells. Once packed, filtration collection conduit 53 is disconnected from lumen-side outlet port 27 and lumen-side inlet port 25 opened. The catalytic zone is maintained at required conditions to ensure enzyme activity. It should be understood that said conditions depend on the particular enzyme being utilized.

Predetermined reactant material is supplied to the reactant/product zone through lumen-side inlet port 25. Said reactant diffuses across the semipermeable membranes of the hollow fibers and contacts a predetermined enzyme present in the catalytic zone. Predetermined product material formed in the catalytic zone by reaction between enzyme and reactant diffuses across said membrane into reactant/product zone and is recovered through lumen-side outlet port 27.

When a hollow fiber biocatalytic reactor was prepared in this manner, the discovery was made that the microbial cells evenly distribute throughout shell-side volume 17 and substantially coat each fiber. Microbial cells essentially fill shell-side volume 17 thereby concentrating available catalyst. Viable microbial cells utilized in the preferred embodiment multiply within shell-side volume 17 further increasing catalyst concentration. The confluent cellular packing of the present invention and cell adhesiveness generally prevent the cells from being separated from the fibers thereby eliminating the need to physically attach the cells to the fiber membrane.

The membrane must be readily permeable to predetermined reactant and product materials but essentially impermeable to the catalytically active microbial cell material contained within shell-side volume 17. Although either anisotropic or nonanisotropic membranes can be used in the present invention, nonanisotropic membranes are preferred.

Use of nonanisotropic fibers minimizes contamination of the membrane with cell debris as can frequently occur with the sponge layer of anisotropic fibers. Use of thin walled, nonanisotropic fibers provides a short diffusional path for both reactant and product. Semipermeable fiber membranes can be engineered to accommodate the required molecular weight cutoff.

By removing culture filtrate when packing cells this invention provides a cell concentration that far surpasses previously reported methods for immobilizing whole microbial cells within the shell of hollow fiber cartridges. The extremely dense packing results in high volume productivity and an unexpected stability on some enzyme systems thus greatly reducing processing cost. However, since the cells are not physically attached or entrapped within the membrane, spent cells can be easily washed from the cartridge by a turbulent flow of wash solution between the two shell-side ports. The cartridge can then be easily repacked with fresh cells.

### EXAMPLE 1

A hollow fiber cartridge was purchased having an outside diameter of 3.5 centimeters and a length of 21 centimeters (Travenol Laboratories, Model 15-11). The cartridge contained approximately 8,100 cupraammonium cellulose fibers with wall thickness of 11 microns and outside diameter of 180 microns. Membrane area totaled approximately 1.1 square meters. The cartridge had a lumen volume of about 75 milliliters and a shell volume of about 125 milliliters.

E. coli cells strain ATCC 11303 known to produce aspartase were grown in a 15 liter culture vessel containing 4.7 g $NaH_2PO_4$, 11.2 g $K_2HPO_4$, 2.6 g

$(NH_4)_2SO_4$, 0.3 mg $FeSO_4$, 0.14 mg $ZnCl_2$, 73.9 mg $MgSO_4$ $7H_2O$, 1.5 mg $CaCl_2$, 10.0 g fumaric acid, 6.5 g glycol, and 1.0 g aspartic acid per liter of medium brought to pH 7.0 with $NH_4OH$. About 160 grams (calculated wet weight) of said cells were packed into the shell of said hollow fiber cartridge according to the above-described procedure yielding an essentially confluent cell packing. The column was maintained at $25^{\circ}C$ during the packing procedure.

A feed solution consisting of 1.5 M ammonium fumarate and 1.0 mM magnesium chloride at pH 8.8 and $37^{\circ}C$ was pumped through the lumen-side inlet port at a controlled rate. Aliquots of lumen effluent were collected, diluted, and assayed to determine the conversion of ammonium fumarate to aspartic acid. Essentially complete conversion occurred up to a flow rate of 25 milliliters of ammonium fumarate solution per minute. Said feed rate was then reduced to 21 milliliters per minute and the conversion level monitored. After 84 days said catalytic bioreactor still maintained a 99 percent conversion level. The catalyst half-life was surprisingly found to be about 136 days. Batch studies of E. coli cells in dilute solution suggest that the expected equivalent half-life of the catalyst in the cartridge should have been less than about 32 days. The annualized productivity of the aspartic acid bioreactor based on the aforementioned data was determined to be approximately 2000 kilograms of aspartic acid per year.

### EXAMPLE 2

The same type of hollow fiber cartridge as described in Example 1 was used for the production of alanine from aspartic acid.

Alcaligenes faecalis cells strain ATCC 25094 known to produce aspartate -decarboxylase were grown in a 15 liter culture vessel containing 0.75 g $KH_2PO_4$, 0.94 g $Na_2HPO_4$, 0.20 g $MgSO_4$, 0.03 g $CaCl_2$, 0.004 g $FeCl_3$, 0.003 g $MnCl_2$, 0.001 g $Na_2MoO_4$, 6.43 g aspartic acid, 6.75 g sodium succinate and 0.669 g $NH_4Cl$ per liter of medium brought to pH 7.0 with NaOH. About 100 grams (calculated wet weight) of said cells were packed into the shell of said hollow fiber cartridge according to the above-described procedure yielding a cell packing of about 80 percent of the shell-side volume.. The column was maintained at 25°C during the packing procedure.

A feed solution consisting of 0.50 M aspartic acid, $3.3 \times 10^{-4}$M sodium pyruvate, $1 \times 10^{-4}$M pyridoxal phosphate and 127 mM sodium acetate buffer at pH 5.5 and 37°C was pumped through the lumen side inlet port at a controlled rate. Aliquots of lumen effluent were collected, diluted, and assayed to determine the conversion of aspartic acid to alanine. A 60% conversion of aspartic acid into alanine occurred up to a flow rate of 4.0 milliliters of aspartic acid solution per minute. Bubbles of carbon dioxide gas, generated as a product of the reaction, eluted with the liquid product stream. The conversion level was monitored for several days. After three days the said biocatalytic reactor maintained a 20% conversion. The catalyst half-life was found to be about 2.8 days. The annualized productivity of the alanine bioreactor based on the aforementioned data was determined to be approximately 35 kilograms of alanine per year.

## EXAMPLE 3

The same type of hollow fiber cartridge as described in Example 1 was used for the production of 6-aminopenicillanic acid from penicillin G.

Escherichia coli cells, strain ATCC 9637, were grown in a 28-liter culture vessel containing 2.0 g phenoxyacetic acid, 20.0 g peptone, 5.0 g yeast extract, 5.0 g sodium L-glutamate, 3.0 g $KH_2PO_4$, 0.2 g $MgSO_4 \cdot 7H_2O$ and 0.2 g $FeCl_3 \cdot 6H_2O$ per liter of aqueous medium brought to pH 7.0 with NaOH. About twenty liters of culture medium containing approximately 7.0 g of E. coli cells per liter were flushed through the cartridge yielding an estimated cell packing of about 140 grams wet weight of cells in a total shell volume of 125 ml. The hollow fiber cartridge was maintained at 30°C during both packing and reaction periods.

A feed solution consisting of 50 mM penicillin G in 10 mM potassium phosphate/borate buffer pH 8.5 was pumped through the lumen side inlet port at a controlled rate of 1.0 ml/min. Aliquots of lumen effluent were collected and assayed to determine the conversion of penicillin G to 6-aminopenicillanic acid. The catalyst halflife in the biocatalytic reactor was found to be about 45 days. The catalyst halflife for the same cells freely suspended in the feed solution described above at 30°C was determined to be only 35 days.

## EXAMPLE 4

The same type of hollow fiber cartridge as described in Example 1 was used for the production of urocanic acid from L-histidine.

Bacillus subtilis cells, strain BSCC 1A273, were grown in a 28-liter culture vessel containing

14.0 g $K_2HPO_4$, 6.0 g $KH_2PO_4$, 0.2 g $MgSO_4$, 4.0 g L-histidine and 1.0 ml of a salt mixture per liter of aqueous medium brought to pH 7.0 with NaOH. The salt mixture consisted of 100 mg $ZnSO_4$, 20 mg $FeCl_3 \cdot 6H_2O$, 10 mg $CuSO_4 \cdot 5H_2O$, 70 mg $MnCl_2 \cdot 4H_2O$, 40 mg $(NH_4)_2Mo_6O_{26} \cdot 4H_2O$, 90 mg $Na_2B_4O_7 \cdot 10H_2O$, 500 mg $CaCl_2$ and 20 mg $CoCl_2 \cdot 6H_2O$ per liter of the aqueous salt mixture. About fifty liters of culture medium containing approximately 1.5 g of B. subtilis cells per liter were flushed through the cartridge yielding an estimated cell packing of about 75 grams wet weight of cells in a total shell volume of 125 ml. The hollow fiber cartridge was maintained at $37^{\circ}C$ during both packing and reaction periods.

A feed solution consisting of 0.2 M L-histidine in 0.1 M diethanolamine pH 9.4 was pumped through the lumen side inlet port at a controlled rate of 4.0 ml/min. Aliquots of lumen effluent were collected and assayed to determine the conversion of L-histidine to urocanic acid. The catalyst halflife in the biocatalytic reactor was found to be about 24 days. The catalyst halflife for the same cells freely suspended in the feed solution described above at $37^{\circ}C$ was determined to be only 20 days.

It should be understood that the above examples illustrate a specific embodiment of the present invention but are not intended to limit the scope of the invention. Rather, one skilled in the art could make minor modifications while not departing from the spirit of the present invention.

CLAIMS:

1.  A biocatalytic reactor comprising a first zone and a second zone separated each from the other at least in part by a semipermeable membrane, said membrane being permeable to predetermined reactant and product materials while impermeable to catalytically active microbial cell material confluently packed in said second zone, said microbial cell material being selected such that when in contact with said reactant said product is formed, means for ingress of said reactant to said first zone and means for egress of said product from said first zone.

2.  The apparatus of Claim 1 wherein said semipermeable membrane is an anisotropic membrane.

3.  The apparatus of Claim 1 wherein sad semipermeable membrane is a nonanisotropic membrane.

4.  The apparatus of Claim 1 wherein said semipermeable membrane is a semipermeable hollow fiber membrane.

5.  The apparatus of Claim 1 wherein said microbial cell material occupies greater than about 50 percent of said second zone volume utilized.

6.  A process for conducting a biocatalytic reaction which comprises passing a predetermined reactant material into a first zone enclosed at least in part by a semipermeable membrane boundary which is permeable to said reactant and a predetermined product of said reaction such that said reactant permeates said membrane and contacts catalytically active microbial cell material confluently packed within a second zone separated from said first zone at least in part by said membrane which is essentially impermeable to said microbial cell material, said contact results in conversion of said reactant to said product, said product permeates said semipermeable membrane and is removed from said first zone.

7. The process of Claim 6 wherein said semipermeable membrane is an anisotropic membrane.

8. The process of Claim 6 wherein said semipermeable membrane is a nonanisotropic membrane.

9. The process of Claim 6 wherein said semipermeable membrane is a semipermeable hollow fiber membrane.

10. The process of Claim 6 wherein said microbial cell material occupies greater than about 50 percent of said second zone volume utilized.

FIG.1.

FIG.2.

FIG.3.